(19) Europäisches Patentamt
European Patent Office
Office européen des brevets

(11) Veröffentlichungsnummer: **0 248 253**
**B1**

(12) # EUROPÄISCHE PATENTSCHRIFT

(45) Veröffentlichungstag der Patentschrift: **26.09.90**

(51) Int. Cl.$^5$: **C 07 D 249/08**

(21) Anmeldenummer: **87107156.9**

(22) Anmeldetag: **18.05.87**

(54) **Verfahren zur Herstellung eines Azol-Derivates.**

(30) Priorität: **31.05.86 DE 3618379**

(43) Veröffentlichungstag der Anmeldung:
**09.12.87 Patentblatt 87/50**

(45) Bekanntmachung des Hinweises auf die Patenterteilung:
**26.09.90 Patentblatt 90/39**

(84) Benannte Vertragsstaaten:
**BE CH DE FR GB IT LI NL**

(56) Entgegenhaltungen:
**EP-A-0 001 414**
**EP-A-0 027 177**
**EP-A-0 076 370**
**EP-A-0 102 543**

(73) Patentinhaber: **BAYER AG**
**D-5090 Leverkusen 1 Bayerwerk (DE)**

(72) Erfinder: **Reiser, Wolf, Dr.**
**Kiebitzweg 12a**
**D-5600 Wuppertal 1 (DE)**
Erfinder: **Stetter, Jörg, Dr.**
**Gellertweg 4**
**D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 248 253 B1

**Beschreibung**

Die Erfindung betrifft ein neues Verfahren zur Herstellung des bekannten 3,3-Dimethyl-1-(4-methoximinomethyl-phenoxy)-1-(1,2,4-triazol-1-yl)-butan-2-ols.

Es ist bereits bekannt, daß sich das fungizid wirksame 3,3-Dimethyl-1-(4-methoximinomethyl-phenoxy)-1-(1,2,4-triazol-1-yl)-butan-2-ol in Form von Diastereomeren-Gemischen nach mehreren Verfahren herstellen läßt (vgl. EP—A—0 076 370). So erhält man 3,3-Dimethyl-1-(4-methoximinomethyl-phenoxy)-1-(1,2,4-triazol-1-yl)-butan-2-ol in Form eines Diastereomeren-Gemisches, wenn man 3,3-Dimethyl-1-brom-1-(4-methoximinomethyl-phenoxy)-butan-2-on mit 1,2,4-Triazol- umsetzt und das dabei entstehende 3,3-Dimethyl-1-(4-methoximinomethyl-phenoxy)-1-(1,2,4-triazol-1-yl)-butan-2-on mit Natriumborhydrid reduziert. Nachteilig an diesem Verfahren ist aber, daß jeweils Diastereomeren-Gemische anfallen, die variierende Mengen an den beiden Diastereomeren (A) und (B) aufweisen. Ferner ist das als Zwischenprodukt benötigte 3,3-Dimethyl-1-brom-1-(4-methoximinomethyl-phenoxy)-butan-2-on relativ kostspielig.

In der EP—A—0 001 414 wird die Herstellung von *reinen* diastereomeren Formen (A) von bestimmten Triazolyl-Derivaten durch stereoselektive Reduktion der entsprechenden Ketone mit Aluminium-isopropylat offenbart. Dabei werden die als Ausgangsstoffe eingesetzten Ketone erhalten, indem man 1-Aryl-1-halogeno-3,3-dimethyl-butan-2-one mit 1,2,4-Triazol zur Reaktion bringt. Eine Synthese dieser Ausgangsstoffe durch Chlorierung von 3,3-Dimethyl-1-(1,2,4-triazol-1-yl)-butan-2-on und anschließende Umsetzung des dabei entstehenden 1-Chlor-3,3-dimethyl-1-(1,2,4-triazol-1-yl)-butan-2-ons mit Phenol-Derivaten wird jedoch nicht beschrieben. Außerdam werden keine Verbindungen erwähnt, in denen eine Methoximino-methyl-Gruppe am Phenyl-Rest enthalten ist.

Es wurde gefunden, daß sich das bekannte 3,3-Dimethyl-1-(4-methoximinomethyl-phenoxy)-1-(1,2,4-triazol-1-yl)-butan-2-ol der Formel

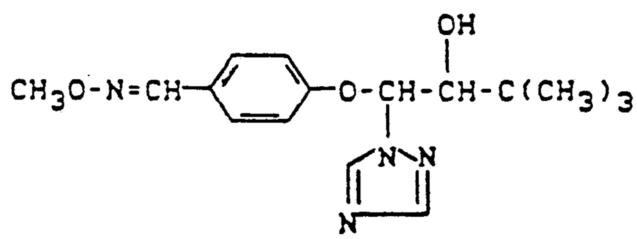

(I)

als kristalline Substanz in Form eines Diastereomeren-Gemisches, das zu 80% aus dem Diastereomeren (A) und zu 20% aus dem Diastereomeren (B) besteht, wobei das Diastereomere (A) für die fungizide Wirkung der Verbindung der Formel (I) entscheidend ist, herstellen läßt, indem man

a) 3,3-Dimethyl-1-(1,2,4-triazol-1-yl)-butan-2-on der Formel

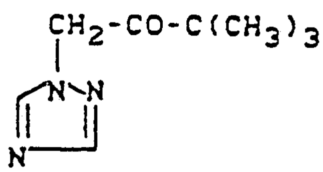

(II)

mit Sulfurylchlorid in Gegenwart von Chlorbenzol bei Temperaturen zwischen 80°C und 130°C umsetzt,

b) dann das dabei entstehende 1-Chlor-3,3-dimethyl-1-(1,2,4-triazol-1-yl)-butan-2-on der Formel

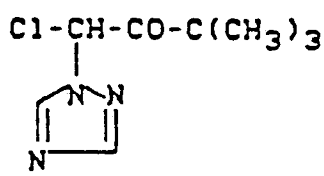

(III)

mit 4-Hydroxybenzaldehyd-O-methyl-oximether der Formel

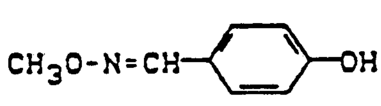

(IV)

in Gegenwart von Isopropanol sowie in Gegenwart von Kaliumhydroxid oder Natriumhydroxid bei Temperaturen zwischen 40°C und 90°C umsetzt und

c) anschließend das so erhaltene 3,3-Dimethyl-1-(4-methoximinomethyl-phenoxy)-1-(1,2,4-triazol-1-yl)-butan-2-on der Formel

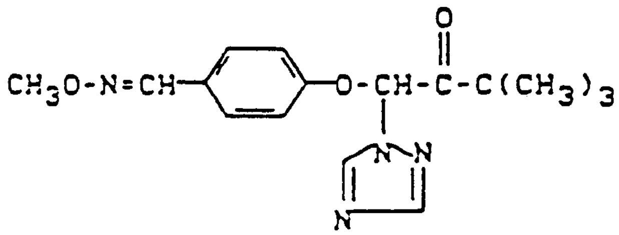

(V)

mit Aluminium-isopropylat in Gegenwart von Isopropanol bei Temperaturen zwischen 80°C und 110°C reduziert.

Überraschenderweise erhält man das 3,3-Dimethyl-1-(4-methoximinomethyl-phenoxy)-1-(1,2,4-triazol-1-yl)-butan-2-ol der Formel (I) nach dem erfindungsgemäßen Verfahren in Form eines Gemisches, in dem das Verhältnis der beiden Diastereomeren reproduzierbar konstant ist, während im Falle des vorbeschriebenen Verfahrens jeweils Gemische entstehen, in denen das Verhältnis der beiden Diastereomeren (A) und (B) zueinander variiert.

Das erfindungsgemäße Verfahren zeichnet sich durch eine Reihe von Vorteilen aus. So sind die benötigen Ausgangsstoffe ohne Schwierigkeiten zu handhaben und auch in größeren Mengen verfügbar. Ferner wird der Einsatz des relativ kostspieligen 3,3-Dimethyl-1-(4-methoximinomethylphenoxy)-butan-2-ons vermieden und die Durchführung des Verfahrens sowie die Aufarbeitung des Reaktionsgemisches bereiten keinerlei Probleme. Von besonderem Vorteil ist außerdem, daß in dem entstehenden Diastereomeren-Gemisch diejenige Komponente, welche für die Wirksamkeit entscheidend ist, in wesentlich größerer Menge vorhanden ist als das weniger wirksame Diastereomere.

Der Verlauf des erfindungsgemäßen Verfahrens kann durch das folgende Formelschema veranschaulicht werden:

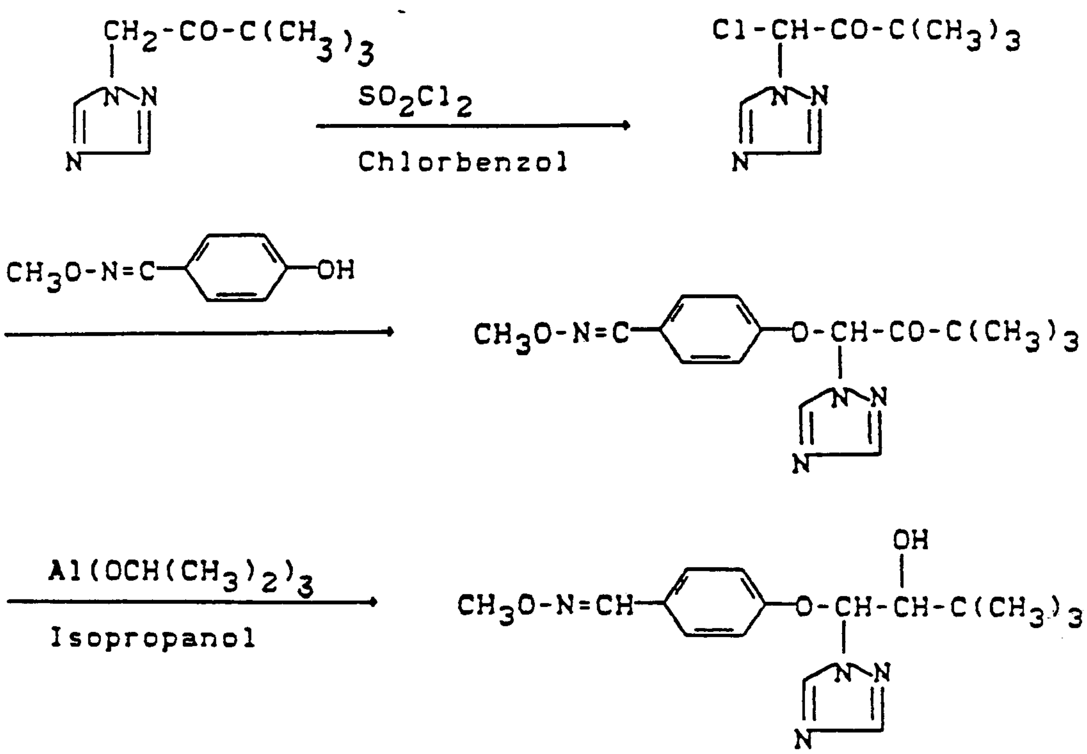


Das bei dem erfindungsgemäßen Verfahren als Ausgangsmaterial benötigte 3,3-Dimethyl-1-(1,2,4-triazol-1-yl)-butan-2-on der Formel (II) ist bekannt (vgl. DE—C—2 431 407).

Bei der Durchführung des erfindungsgemäßen Verfahrens wird der als Reaktionskomponente (zweite Stufe) benötigte 4-Hydroxy-benzaldehyd-O-methyl-oximether der Formel (IV) entweder in Gegenwart von Kaliumhydroxid oder Natriumhydroxid eingesetzt.

Der 4-Hydroxybenzaldehyd-O-methyl-oximether ist bekannt (vgl. EP—A—0 076 370).

Das erfindungsgemäße Verfahren wird im allgemeinen unter Normaldruck durchgeführt. Es ist jedoch auch möglich, unter erhöhtem oder vermindertem Druck zu arbeiten.

Bei der Durchführung des erfindungsgemäßen Verfahrens setzt man in der ersten Stufe auf 1 Mol an 3,3-Dimethyl-1-(1,2,4-triazol-1-yl)-butan-2-on der Formel (II) im allgemeinen 0,5 bis 2 Mol, vorzugsweise 1 bis 1,2 Mol an Sulfurylchlorid ein. In der zweiten Stufe setzt man auf 1 Mol an 1-Chlor-3,3-dimethyl-1-(1,2,4-triazol-1-yl)-butan-2-on der Formel (III) im allgemeinen 0,9 bis 1,5 Mol, vorzugsweise 1 bis 1,1 Mol an 4-Hydroxybenzaldehyd-O-methyl-oximether der Formel (IV) bzw. an dessen Salz sowie gegebenenfalls 0,9 bis 1,5 Mol an Säurebindemittel ein.

In der dritten Stufe setzt man auf 1 Mol an 3,3-Dimethyl-1-(4-methoximinomethyl-phenoxy)-1-(1,2,4-triazol-1-yl)-butan-2-on der Formel (V) im allgemeinen 0,5 bis 2 Mol an Aluminium-isopropylat ein. Die Aufarbeitung erfolgt jeweils nach üblichen Methoden.

Bei der Durchführung des erfindungsgemäßen Verfahrens erhält man 3,3-Dimethyl-1-(4-methoximinomethyl-phenoxy)-1-(1,2,4-triazol-1-yl)-butan-2-ol der Formel (I) in Form eines Diastereomeren-Gemisches, das zu 80% aus dem Diastereomeren (A) und zu 20% aus dem Diastereomeren (B) besteht, wobei das Diastereomere (A) für die fungizide Wirkung der Verbindung der Formel (I) entscheidend ist.

Das erfindungsgemäße Verfahren wird durch die folgenden Beispiele veranschaulicht.

Beispiel 1

a) 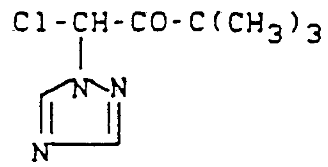

In einem Dreihalskolben, der mit Tropftrichter, Rührer und Thermometer versehen ist, wird ein Gemisch aus 173,2 g 3,3-Dimethyl-1-(1,2,4-triazol-1-yl)-butan-2-on auf 100°C erhitzt. Danach werden innerhalb von 4 Stunden unter Rühren 148,5 g Sulfurylchlorid zugetropft. Es wird noch 30 Minuten bei 100°C nachgerührt, dann wird das Reaktionsgemisch auf Raumtemperatur abgekühlt und mit Wasser versetzt. Man neutralisiert das Gemisch durch Zugabe von gesättigter, wäßriger Natriumhydrogencarbonat-Lösung und trennt die Phasen. Die organische Phase wird zweimal mit Wasser gewaschen und dann unter vermindertem Druck eingeengt. Man erhält 200,4 g eines Produktes, das gemäß Gaschromatogramm zu 92,5% aus 1-Chlor-3,3-dimethyl-1-(1,2,4-triazol-1-yl)-butan-2-on besteht. Danach errechnet sich eine Ausbeute von 91,9% der Theorie.

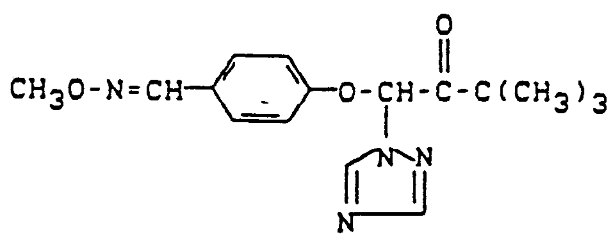

In ein Gemisch aus 136 g 4-Hydroxybenzaldehyd-O-methyl-oximether und 205 ml Isopropanol werden unter Rühren 33,2 g Natriumhydroxid gegeben. Man erwärmt das Reaktionsgemisch auf 40—50°C und läßt eine Lösung von 194,2 g 1-Chlor-3,3-dimethyl-1-(1,2,4-triazol-1-yl)-butan-2-on in 100 ml Isopropanol hinzutropfen. Danach wird zunächst 3 Stunden bei 40—45°C nachgerührt, dann auf Raumtemperatur abgekühlt, mit weiteren 350 ml Wasser versetzt und auf 09—5°C abgekühlt. Es wird 1 Stunde bei 0—5°C nachgerührt. Anschließend wird der entstandene Niederschlag abfiltriert, mit 100 ml eines zu gleichen Teilen aus Isopropanol und Wasser bestehenden Gemisches gewaschen und dann getrocknet. Man erhält auf diese Weise 207 g eines Produktes, das gemäß HPLC-Analyse zu 97,4% aus 3,3-Dimethyl-1-(4-methoximinomethyl-phenoxy)-1-(1,2,4-triazol-1-yl)-butan-2-on besteht. Die Ausbeute errechnet sich danach zu 76,9% der Theorie.

Fp. 92—92,5°C.

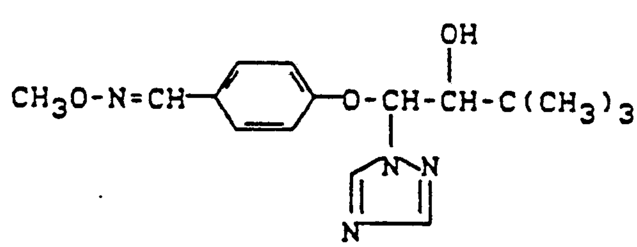

In einen 3 l Rührautoklaven werden 323,8 g 3,3-Dimethyl-1-(4-methoximino-methyl-phenoxy)-1-(1,2,4-triazol-1-yl)-butan-2-on, 600 ml Isopropanol und 102 g Aluminiumisopropylat gegeben. Der Autoklav wird verschlossen und 6 Stunden auf 110°C erhitzt, wobei sich ein Druck von 3—3,5 bar (SI-Einheit) einstellt.

Nach dem Abkühlen des Reaktionsgemisches auf Raumtemperatur wird das Verdünnungsmittel abdestilliert und der Rückstand in 600 ml Cyclohexan aufgenommen. Die entstehende Suspension wird auf 50°C erwärmt und tropfenweise mit 380 ml Wasser und 77 g konzentrierter Schwefelsäure versetzt. Die Phasen werden bei 65°C getrennt, und die organische Phase wird zweimal mit je 500 ml Wasser extrahiert. Nach Phasentrennung bei 65°C wird abgekühlt und der ausgefallene Niederschlag abfiltriert und getrocknet. Man erhält auf diese Weise 296,8 g eines kristallinen Produktes, das zu 97,4% aus 3,3-Dimethyl-1-(4-methoximino-methylphenoxy)-1-(1,2,4-triazol-1-yl)-butan-2-ol besteht. Die Ausbeute errechnet sich danach zu 90,9% der Theorie.

**Patentanspruch**

Verfahren zur Herstellung von 3,3-Dimethyl-1-(4-methoximinomethyl-phenoxy)-1-(1,2,4-triazol-1-yl)-butan-2-ol der Formel

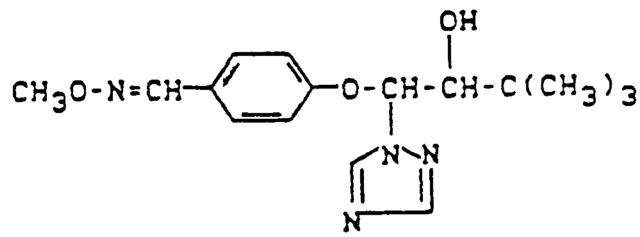

(I)

als kristalline Substanz in Form eines Diastereomeren-Gemisches, das zu 80% aus dem Diastereomeren (A) und zu 20% aus dem Diastereomeren (B) besteht, wobei das Diastereomere (A) für die fungizide Wirkung der Verbindung der Formel (I) entscheidend ist, dadurch gekennzeichnet, daß man

a) 3,3-Dimethyl-1-(1,2,4-triazol-1-yl)-butan-2-on der Formel

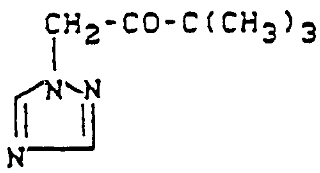

(II)

mit Sulfurylchlorid in Gegenwart von Chlorbenzol bei Temperaturen zwischen 80°C und 130°C umsetzt,

b) dann das dabei entstehende 1-Chlor-3,3-dimethyl-1-(1,2,4-triazol-1-yl)-butan-2-on der Formel

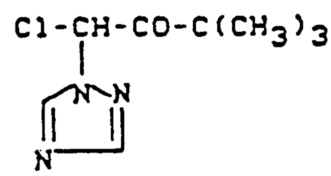

(III)

mit 4-Hydroxybenzaldehyd-O-methyl-oximether der Formel

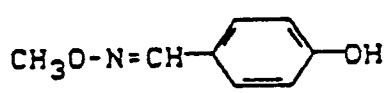

(IV)

in Gegenwart von Isopropanol sowie in Gegenwart von Kaliumhydroxid oder Natriumhydroxid bei Temperaturen zwischen 40°C und 90°C umsetzt und

c) anschließend das so erhaltene 3,3-Dimethyl-1-(4-methoximinomethyl-phenoxy)-1-(1,2,4-triazol-1-yl)-butan-2-on der Formel

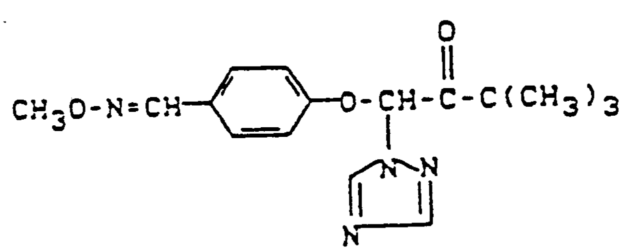

(V)

mit Aluminium-isopropylat in Gegenwart von Isopropanol bei Temperaturen zwischen 80°C und 110°C reduziert.

## Revendication

Procédé de production de 3,3-diméthyl-1-(4-méthoximinométhyl-phénoxy)-1-(1,2,4-triazole-1-yl)-butane-2-ol de formule

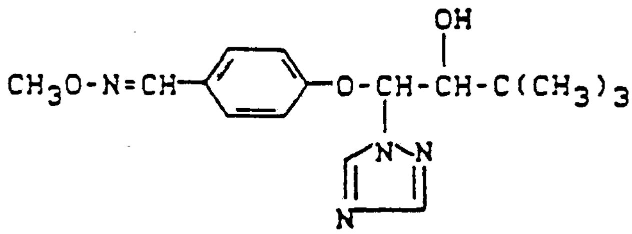

(I)

comme substance cristalline sous forme d'un mélange de diastéréo-isomères, qui est constitué à 80% du diastéréo-isomère (A) et à 20% du diastéréo-isomère (B), le diastéréo-isomère (A) étant déterminant pour l'action fongicide du composé de formule (I), caractérisé en ce que

(a) on fait réagir la 3,3-diméthyl-1-(1,2,4-triazole-1-yl)-butane-2-one de formule

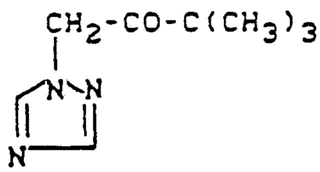

(II)

avec le chlorure de sulfuryle en présence de chlorobenzène à des températures comprises entre 80 et 130°C,

(b) on fait ensuite réagir la 1-chloro-3,3-diméthyl-1-(1,2,4-triazole-1-yl)-butane-2-one ainsi produite de formule

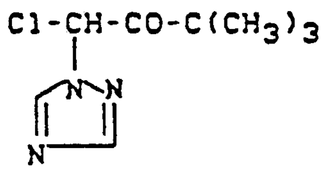

(III)

avec le O-méthyloxime-éther de 4-hydroxybenzaldéhyde de formule

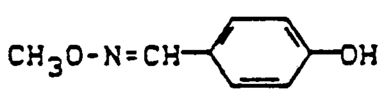

(IV)

en présence d'isopropanol ainsi qu'en présence d'hydroxyde de potassium ou d'hydroxyde de sodium à des températures comprises entre 40 et 90°C et

(c) on réduit ensuite la 3,3-diméthyl-1-(4-méthoximinométhyl-phénoxy)-1-(1,2,4-triazole-1-yl)-butane-2-one ainsi obtenue de formule

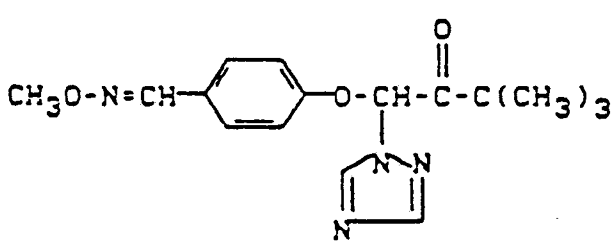

(V)

avec l'isopropylate d'aluminium en présence d'isopropanol à des températures comprises entre 80°C et 110°C.

**Claim**

Process for the preparation of 3,3-dimethyl-1-(4-methoximinomethyl-phenoxy)-1-(1,2,4-triazol-1-yl)-butan-2-ol of the formula

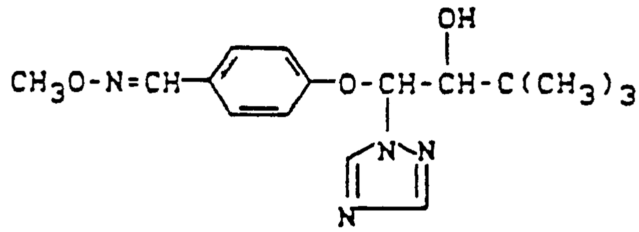

(I)

as a crystalline substance in the form of a diastereomer mixture which consists to the extent of 80% of diastereomer (A) and to the extent of 20% of diastereomer (B), diastereomer (A) being decisive for the fungicidal action of the compound of the formula (I), characterized in that

a) 3,3-dimethyl-1-(1,2,4-triazol-1-yl)-butan-2-one of the formula

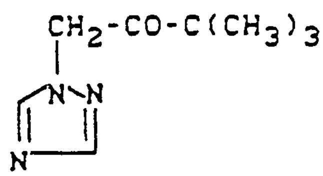

(II)

is reacted with sulphuryl chloride in the presence of chlorobenzene at temperatures between 80°C and 130°C,

b) the 1-chloro-3,3-dimethyl-1-(1,2,4-triazol-1-yl)-butan-2-one of the formula

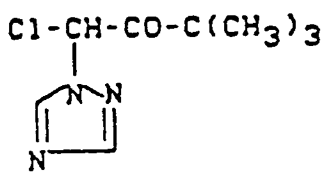

(III)

produced in this reaction is then reacted with 4-hydroxybenzaldehyde oxime O-methyl ether of the formula

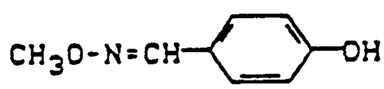

(IV)

in the presence of isopropanol and in the presence of potassium hydroxide or sodium hydroxide at temperatures between 40°C and 90°C, and

c) the 3,3-dimethyl-1-(4-methoximinomethyl-phenoxy)-1-(1,2,4-triazol-1-yl)-butan-2-one of the formula

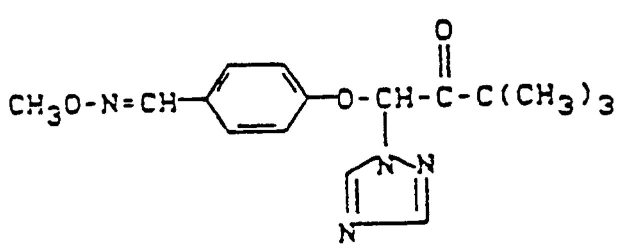

(V)

thus obtained is subsequently reduced using aluminium isopropylate in the presence of isopropanol at temperatures between 80°C and 110°C.